# EUROPEAN PATENT APPLICATION

(11) **EP 1 566 443 A1**
(43) Date of publication of application: **24.08.2005**
(21) Application number: 04004049.5
(22) Date of filing: 23.02.2004
(51) Int. Cl.: C12N 15/82, A01H 4/00

(54) **Improved transformation of brassica species**

(71) Applicant: SunGene GmbH & Co.KgaA, 06466 Gatersleben (DE)
(72) Inventor: Kunze, Irene, Dr., 06466 Gatersleben (DE); Heim, Ute, Dr., 06466 Gatersleben (DE); Herbers, Karin, Dr., 06484 Quedlinburg (DE)
(74) Representative: Heistracher, Elisabeth

(57) **Abstract**

SUMMARY OF THE INVENTION

the invention related to a method for producing a transgenic Brassica plant comprising the steps of:
(a) isolating cotyledonary petioles from germinated seeds of a Brassica plant,
(b) treating said petioles with an Agrobacterium solution,
(c) transferring said treated petioles on a co-cultivation medium comprising at least one plant growth factor solidified with an agar, said agar characterized by an electroendosmosis value (Mr) of below 0.5, a content of calcium ions of less then 1000 ppm, and a content of magnesium ions of less then 500 ppm, and culturing said petioles on said co-cultivation medium for about 1 to about 5 days,
(d) transferring said co-cultivated petioles on a regeneration and selection medium comprising at least one plant growth factor,
(e) cultivating said petioles on said co-cultivation and regeneration medium until shoots are induced and developed from said petioles and isolating said shoots from said petioles,
(f) transferring said isolated shoots on a shoot elongation and regeneration medium, wherein the agar content in said medium is between about 0.3% and about 0.6% (w/w) agar, and wherein said medium comprises between about 1% and 2.5% (w/w) sucrose,
(g) cultivating said isolated shoots on said shoot elongation and regeneration medium until said shoots have formed roots, and further regenerating the so derived plantlets into mature plants, which comprise inserted into their genome a T-DNA comprising a selectable marker gene and - optionally - an additional plant expression cassette for an agronomically valuable trait.

## Description

### BACKGROUND OF THE INVENTION

### Field of the Invention

The present invention relates to improved methods for the incorporation of DNA into the genome of a Brassica plant, preferably a Brassica napus plant, by means of Agrobacterium-mediated transformation.

### Background

Brassica species are widely used as a source of protein, oil, condiments and chemical feedstock. Significant effort has been expended to improve the quality of cultivated Brassica species by conventional plant breeding, and a number of major successes are recorded. The methods of conventional plant breeding have been limited, however, to the movement of genes and traits from members of the genus Brassica to others of the same genus and, in a few notable examples, "wide crosses" from other closely related genera.

The development of a method for introducing foreign genes into Brassica species greatly enhanced the range of traits which could be imparted to Brassica oilseeds and vegetables. In order to obtain a system for useful gene introduction into Brassica species, a number of obstacles had to be overcome. These include optimization of regeneration to whole plants of the target tissue, definition of the conditions (e.g., time, bacterial concentration, and media) for the co-cultivation of the Brassica cells and Agrobacterium cells, and establishing an appropriate selection protocol.

Brassica species have been widely investigated for regenerability of tissue explants. Both Brassica napus and Brassica oleracea show shoot regeneration from a variety of tissues including hypocotyls (Dietert 1982), leaf callus (Stringham 1979), roots (Lazzeri 1984), and leaf and stem protoplasts (Li 1982; Glimelius 1984).

EP-A1 270 615 discloses a method for transforming Brassica cells by co-cultivating Brassica cells with A. tumefaciens comprising a T-DNA comprising the nptII selection marker. The target tissue employed therein are either leaf disks or hypocotyl tissue. The co-cultivated tissue is then transferred to callus inducing media (containing an auxin) and selected on kanamycin medium. Moloney (1989) describes a method similar to the method described in EP-A1 270 615 but using cotyledonary petioles as target tissue. De Block (1989) describes a method similar to the method described in EP-A1 270 615, also based on hypocotyl tissue, but using modified media. A comparison of the details of the methods known in the prior art is given below in the Examples.

Although some of the problems linked to the transformation of Brassica species has been overcome by the methods described in the art, there is still a significant need for improvement, since all methods known so far have only a low to moderate transformation and - especially - regeneration efficiency. Therefore, it was the objective of the present invention to provide an improved method having higher overall efficiency in the process of generation of transgenic Brassica plants. This objective is solved by the present invention.

### SUMMARY OF THE INVENTION

Accordingly a first embodiment of the invention related to a method for producing a transgenic Brassica plant comprising the steps of:
(a) isolating cotyledonary petioles from germinated seeds of a Brassica plant,
(b) treating said petioles with an Agrobacterium solution, wherein said Agrobacterium comprises a T-DNA comprising a selectable marker gene and - optionally - an additional plant expression cassette for an agronomically valuable trait,
(c) transferring said treated petioles on a co-cultivation medium comprising at least one plant growth factor solidified with an agar, said agar characterized by an electroendosmosis value (Mr) of below 0.5, a content of calcium ions of less then 1000 ppm, and a content of magnesium ions of less then 500 ppm, and culturing said petioles on said co-cultivation medium for about 1 to about 5 days,
(d) transferring said co-cultivated petioles on a regeneration and selection medium comprising at least one plant growth factor, at least one antibiotic suitable to inhibit Agrobacterium growth, and at least one compound which in combination with the selectable marker gene of (a) allows for identification and/or selection of plant cells, organs or plants comprising said selectable marker gene, said medium solidified with an agar, said agar characterized by an electroendosmosis value (Mr) of below 0.5, a content of calcium ions of less then 1000 ppm, and a content of magnesium ions of less then 500 ppm,
(e) cultivating said petioles on said co-cultivation and regeneration medium until shoots are induced and developed from said petioles and isolating said shoots from said petioles,
(f) transferring said isolated shoots on a shoot elongation and regeneration medium comprising at least one plant growth factor and a compound which in combination with the selectable marker gene of (a) allows for identification and/or selection of plant cells, organs or plants comprising said selectable marker gene, said medium solidified with an agar, said agar characterized by an electroendosmosis value (Mr) of below 0.5, a content of calcium ions of less then 1000 ppm, and a content of magnesium ions of less then 500 ppm, wherein the agar content in said medium is between about 0.3% and about 0.6% (w/w) agar, and wherein said medium comprises between about 1% and 2.5% (w/w) sucrose,
(g) cultivating said isolated shoots on said shoot elongation and regeneration medium until said shoots have formed roots, and further regenerating the so derived plantlets into mature plants, which comprise inserted into their genome a T-DNA comprising a selectable marker gene and - optionally - an additional plant expression cassette for an agronomically valuable trait.

Critical parameters are the type and content of the agar used as solidifier. As demonstrated in the examples below, this is of eminent influence on the regeneration efficiency.

Preferably, the agar content in said medium is between about 0.3% and about 0.6% (w/w) agar, more preferably between about 0.5% (w/w). The agar is characterized by a low ion content (especially magnesium and calcium ions). Preferably, the content of calcium ions is less then 1000 ppm, more preferably less then 500 ppm, most preferably less then 200 ppm. Preferably, the content of magnesium ions less then 500 ppm, more preferably less then 250 ppm, most preferably less then 100 ppm. The agar is furthermore characterized by a low electroendosmotic value (Mr), which is preferably lower then 0.5, preferably lower then 0.4.

It is further of critical importance to keep the sucrose level (or its equivalent in caloric value) below about 2.5% (w/w), preferably between about 1 and about 2.5% (w/w), more preferably between about 1.5 and about 2.2 % (w/w), most preferably at about 2% (w/w). This is lower then the normal carbon-source content employed in plant cell culture medium.

However, during the germination step it is preferred to have a sucrose content of about 3% (w/w) (instead of 2% as disclosed in the art). The resulting seedlings are smaller but significantly more regenerable. The reduction of sucrose in the shoot elongation / rooting step seems to stimulate shoot elongation.

In another preferred embodiment of the invention the employed Agrobacterium strain is not a supervirulent strain. Preferably, the Agrobacterium strain is a nopaline-type strain.

In another preferred embodiment of the invention, the cotyledonary petioles are isolated from seedlings germinated for not longer than 5 days. Preferably from seedlings germinated for about 4 days under low light condition (20 - 80 µM/m²s) at 24°C.

In another preferred embodiment, the media of at least one of step (c), (d), and/or (e) comprises between about 1 mg/l and about 10 mg/l 6-benzylaminopurine (BAP).

It is furthermore especially preferred, that the media of at least one of step (c), (d), and/or (e) comprises between about 0.1 mg/l and about 2 mg/l Gibberellic acid (GA3).

In another preferred embodiment of the invention, the media of at least one of step (f) and/or (g) comprises between about 0.001 mg/l and about 0.1 mg/l 6-benzylaminopurine (BAP) and between about 0.01 mg/l and about 1 mg/l indole butyric acid (IBA).

In another preferred embodiment, the media employed during the transformation procedure do not comprise PVP (poly-vinylpyrrolidone).
The method of the invention is distinguished from the methods described in the art by at least the following features:
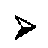 The use of Oxoid™ agar instead of Phytoagar™ (as used in the prior art) in certain concentrations resulted in an significantly improved recovery and transformation rate.
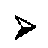 De Block (1989) is using hypocotyl target tissue instead of cotyledonary petioles.
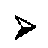 A combination of BAP with GA3 is used during co-cultivation, selection and regeneration, which is used neither in EP 270 615 nor in Moloney (1989).
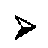 De Block (1989) is using PVP (polyvinylpyrrolidone) in the regeneration and selection step. This has proven to be of disadvantage and seems to stimulate unwanted Agrobacteria growth.

### GENERAL DEFINITIONS

Abbreviations: BAP - 6-benzylaminopurine; 2,4-D - 2,4-dichlorophenoxyacetic acid; MS - Murashige and Skoog medium; NAA - 1-naphtaleneacetic acid; MES, 2-(N-morpholino-ethanesulfonic acid, IBA indole butyric acid; Kan: Kanamycin sulfate; GA3 - Gibberellic acid; Timentin™: ticarcillin disodium / clavulanate potassium.

It is to be understood that this invention is not limited to the particular methodology, protocols, cell lines, plant species or genera, constructs, and reagents described as such. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth.

The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent up or down (higher or lower).

As used herein, the word "or" means any one member of a particular list and also includes any combination of members of that list.

The term "nucleic acid" refers to deoxyribonucleotides or ribonucleotides and polymers or hybrids thereof in either single- or double-stranded, sense or antisense form.

Unless otherwise indicated, a particular nucleic acid sequence also implicitly encompasses conservatively modified variants thereof (e. g., degenerate codon substitutions) and complementary sequences, as well as the sequence explicitly indicated. The term "nucleic acid" is used interchangeably herein with "gene", "cDNA, "mRNA", "oligonucleotide," and "polynucleotide".

The phrase "nucleic acid sequence" as used herein refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid which is transcribed and translated in a sequence-specific manner to produce into a particular polypeptide or protein when placed under the control of appropriate regulatory sequences. The coding region is said to encode such a polypeptide or protein.

The term "antisense" is understood to mean a nucleic acid having a sequence complementary to a target sequence, for example a messenger RNA (mRNA) sequence the blocking of whose expression is sought to be initiated by hybridization with the target sequence.

The term "sense" is understood to mean a nucleic acid having a sequence which is homologous or identical to a target sequence, for example a sequence which binds to a protein transcription factor and which is involved in the expression of a given gene. According to a preferred embodiment, the nucleic acid comprises a gene of interest and elements allowing the expression of the said gene of interest.

The term "gene" refers to a coding region operably joined to appropriate regulatory sequences capable of regulating the expression of the polypeptide in some manner. A gene includes untranslated regulatory regions of DNA (e. g., promoters, enhancers, repressors, etc.) preceding (upstream) and following (downstream) the coding region (open reading frame, ORF) as well as, where applicable, intervening sequences (i.e., introns) between individual coding regions (i.e., exons).

As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5'-side by the nucleotide triplet "ATG" which encodes the initiator methionine and on the 3'-side by one of the three triplets which specify stop codons (i.e., TAA, TAG, TGA). In addition to containing introns, genomic forms of a gene may also include sequences located on both the 5'- and 3'-end of the sequences which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers which control or influence the transcription of the gene. The 3'-flanking region may contain sequences which direct the termination of transcription, posttranscriptional cleavage and polyadenylation.

The terms "polypeptide", "peptide", "oligopeptide", "polypeptide", "gene product", "expression product" and "protein" are used interchangeably herein to refer to a polymer or oligomer of consecutive amino acid residues.

The term "isolated" as used herein means that a material has been removed from its original environment. For example, a naturally occurring polynucleotide or polypeptide present in a living animal is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment.

The term "wild-type", "natural" or of "natural origin" means with respect to an organism, polypeptide, or nucleic acid sequence, that said organism is naturally occurring or available in at least one naturally occurring organism which is not changed, mutated, or otherwise manipulated by man.

The term "transgenic" or "recombinant" as used herein (e.g., with regard to a Brassica cells or plants) is intended to refer to cells and/or plants that have incorporated exogenous genes or DNA sequences, including but not limited to genes or DNA sequences which are perhaps not normally present, genes not normally transcribed and translated ("expressed") in a given cell type, or any other genes or DNA sequences which one desires to introduce into the non-transformed cell and/or plant, such as genes which may normally be present in the non-transformed cell and/or plant but which one desires to have altered expression. Preferably, the term "recombinant" with respect to nucleic acids as used herein means that the nucleic acid is covalently joined and adjacent to a nucleic acid to which it is not adjacent in its natural environment. "Recombinant" polypeptides or proteins refer to polypeptides or proteins produced by recombinant DNA techniques, i. e., produced from cells transformed by an exogenous recombinant DNA construct encoding the desired polypeptide or protein. Recombinant nucleic acids and polypeptide may also comprise molecules which as such does not exist in nature but are modified, changed, mutated or otherwise manipulated by man.

A "recombinant polypeptide" is a non-naturally occurring polypeptide that differs in sequence from a naturally occurring polypeptide by at least one amino acid residue. Preferred methods for producing said recombinant polypeptide and/or nucleic acid may comprise directed or non-directed mutagenesis, DNA shuffling or other methods of recursive recombination.

The terms "heterologous nucleic acid sequence" or "heterologous DNA" are used interchangeably to refer to a nucleotide sequence which is ligated to a nucleic acid sequence to which it is not ligated in nature, or to which it is ligated at a different location in nature. Heterologous DNA is not endogenous to the cell into which it is introduced, but has been obtained from another cell. Generally, although not necessarily, such heterologous DNA encodes RNA and proteins that are not normally produced by the cell into which it is expressed.

The "efficiency of transformation" or "frequency of transformation" as used herein can be measured by the number of transformed cells (or transgenic organisms grown from individual transformed cells) that are recovered under standard experimental conditions (i.e. standardized or normalized with respect to amount of cells contacted with foreign DNA, amount of delivered DNA, type and conditions of DNA delivery, general culture conditions etc.) For example, when isolated petioles are used as starting material for transformation, the frequency of transformation can be expressed as the number of transgenic shoots (or resulting plant lines) obtained per transformed petiole.

The term "cell" refers to a single cell. The term "cells" refers to a population of cells. The population may be a pure population comprising one cell type. Likewise, the population may comprise more than one cell type. In the present invention, there is no limit on the number of cell types that a cell population may comprise. The cells may be synchronize or not synchronized, preferably the cells are synchronized.

The term "chromosomal DNA" or "chromosomal DNA-sequence" is to be understood as the genomic DNA of the cellular nucleus independent from the cell cycle status. Chromosomal DNA might therefore be organized in chromosomes or chromatids, they might be condensed or uncoiled. An insertion into the chromosomal DNA can be demonstrated and analyzed by various methods known in the art like e.g., PCR analysis, Southern blot analysis, fluorescence in situ hybridization (FISH), and in situ PCR.
The term "structural gene" as used herein is intended to mean a DNA sequence that is transcribed into mRNA which is then translated into a sequence of amino acids characteristic of a specific polypeptide.

The term "expression" refers to the biosynthesis of a gene product. For example, in the case of a structural gene, expression involves transcription of the structural gene into mRNA and - optionally - the subsequent translation of mRNA into one or more polypeptides.

The term "transformation" includes introduction of genetic material into plant cells, preferably resulting in chromosomal integration and stable heritability through meiosis. Transformation also includes introduction of genetic material into plant cells in the form of plant viral vectors involving epichromosomal replication and gene expression which may exhibit variable properties with respect to meiotic stability.

The term "expression cassette" or "expression construct" as used herein is intended to mean the combination of any nucleic acid sequence to be expressed in operable linkage with a promoter sequence and - optionally - additional elements (like e.g., terminator and/or polyadenylation sequences) which facilitate expression of said nucleic acid sequence.

The term "promoter" as used herein is intended to mean a DNA sequence that directs the transcription of a DNA sequence (e.g., a structural gene). Typically, a promoter is located in the 5'-region of a gene, proximal to the transcriptional start site of a structural gene. If a promoter is an inducible promoter, then the rate of transcription increases in response to an inducing agent. In contrast, the rate of transcription is not regulated by an inducing agent if the promoter is a constitutive promoter. Also, the promoter may be regulated in a tissue-specific or tissue preferred manner such that it is only active in transcribing the associated coding region in a specific tissue type(s) such as leaves, roots or meristem.

The term "operable linkage" or "operably linked" is to be understood as meaning, for example, the sequential arrangement of a regulatory element (e.g. a promoter) with a nucleic acid sequence to be expressed and, if appropriate, further regulatory elements (such as e.g., a terminator) in such a way that each of the regulatory elements can ful-fill its intended function to allow, modify, facilitate or otherwise influence expression of said nucleic acid sequence. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. Operable linkage, and an expression cassette, can be generated by means of customary recombination and cloning techniques as described (e.g., in Maniatis 1989; Silhavy 1984; Ausubel 1987; Gelvin 1990). However, further sequences which, for example, act as a linker with specific cleavage sites for restriction enzymes, or as a signal peptide, may also be positioned between the two sequences. The insertion of sequences may also lead to the expression of fusion proteins. Preferably, the expression cassette, consisting of a linkage of promoter and nucleic acid sequence to be expressed, can exist in a vector-integrated form and be inserted into a plant genome, for example by transformation.

### DETAILED DESCRIPTION OF THE INVENTION

Accordingly a first embodiment of the invention related to a method for producing a transgenic Brassica plant comprising the steps of:
(a) isolating cotyledonary petioles from germinated seeds of a Brassica plant,
(b) treating said petioles with an Agrobacterium solution, wherein said Agrobacterium comprises a T-DNA comprising a selectable marker gene and - optionally - an additional plant expression cassette for an agronomically valuable trait,
(c) transferring said treated petioles on a co-cultivation medium comprising at least one plant growth regulator solidified with an agar, said agar characterized by an electroendosmosis value (Mr) of below 0.5, a content of Calcium ions of less then 1000 ppm, and a content of Magnesium ions of less then 500 ppm, and culturing said petioles on said co-cultivation medium for about 1 to about 5 days,
(d) transferring said co-cultivated petioles on a regeneration and selection medium comprising at least one plant growth regulator, at least one antibiotic suitable to inhibit Agrobacterium growth, and at least one compound which in combination with the selectable marker gene of (a) allows for identification and/or selection of plant cells, organs or plants comprising said selectable marker gene, said medium solidified with an agar, said agar characterized by an electroendosmosis value (Mr) of below 0.5, a content of calcium ions of less then 1000 ppm, and a content of magnesium ions of less then 500 ppm,
(e) cultivating said petioles on said co-cultivation and regeneration medium until shoots are induced and developed from said petioles and isolating said shoots from said petioles,
(f) transferring said isolated shoots on a shoot elongation and regeneration medium comprising at least one plant growth regulator and a compound which in combination with the selectable marker gene of (a) allows for identification and/or selection of plant cells, organs or plants comprising said selectable marker gene, said medium solidified with an agar, said agar characterized by an electroendosmosis value (Mr) of below 0.5, a content of calcium ions of less then 1000 ppm, and a content of magnesium ions of less then 500 ppm, wherein the agar content in said medium is between about 0.3% and about 0.6% (w/w) agar, and wherein said medium comprises between about 1% and 2.5% (w/w) sucrose,
(g) cultivating said isolated shoots on said shoot elongation and regeneration medium until said shoots have formed roots, and further regenerating the so derived plantlets into mature plants, which comprise inserted into their genome a T-DNA comprising a selectable marker gene and - optionally - an additional plant expression cassette for an agronomically valuable trait.

Any Brassica species can be used in the practice of the present invention such as B. napus (rape seed and rutabaga), B. oleraceae (cabbage, broccoli, brussel sprouts and other oleracea varieties), B. juncea (Indian mustard), B. campestris (turnip rape), and the like. Preferred is Brassica napus and all varieties thereof.

Cotyledonary petioles are employed as target tissue for the method of the invention. In another preferred embodiment of the invention, the cotyledonary petioles are isolated from seedlings germinated for not longer then 5 days. Preferably from seedlings germination for about 4 days under low light condition (20 - 80 µM/m²s) at 24°C.

As demonstrated below, one critical parameter is the type and content of the agar used as solidifier of the media employed during transformation and especially regeneration. This is of eminent influence on the regeneration efficiency.

Preferably, the agar content in said medium is between about 0.3% and about 0.6% (w/w) agar, more preferably between about 0.5% (w/w). The agar is characterized by a low ion content (especially magnesium and calcium ions). Preferably, the content of Calcium ions is less then 1000 ppm, more preferably less then 500 ppm, most preferably less then 200 ppm. Preferably, the content of magnesium ions less then 500 ppm, more preferably less then 250 ppm, most preferably less then 100 ppm. The agar is furthermore characterized by a low electroendosmotic value (Mr), which is preferably lower then 0.5, preferably lower then 0.4.

It is further of critical importance to keep the sucrose level (or its equivalent in caloric value) below about 2.5% (w/w), preferably between about 1 and about 2.5% (w/w), more preferably between about 1.5 and about 2.2 % (w/w), most preferably at about 2% (w/w). This is lower then the normal carbon-source content employed in plant cell culture medium.

However, during the germination step it is preferred to have a sucrose content of about 3% (w/w) or higher (up to 10% (w/w)) (instead of 2% as disclosed in the art). The resulting seedlings are smaller but significantly more regenerable. The reduction of sucrose in the shoot elongation / rooting step seems to stimulate shoot elongation. Preferably the sucrose content is between about 3% (w/w) and about 5% (w/w).

The T-DNA introduced into the Brassica species of the invention genetic is introduced by means of Agrobacterium-mediated DNA transfer. The term "Agrobacterium" as used herein means all species of the Agrobacterium family (including Agrobacterium tumefaciens and Agrobacterium rhizogenes). Preferably, transformation is realized utilizing strains of Agrobacterium tumefaciens or Agrobacterium rhizogenes. The principles of plant transformation by means of Agrobacterium-mediated DNA transfer are well known in the art (Horsch 1985).

The Agrobacterium strain will include a DNA construct (e.g., a plasmid) comprising a T-DNA which comprises at least one selectable marker gene and - optionally - an additional plant expression cassette for an agronomically valuable trait. As a result of the Agrobacterium mediated transfer, said T-DNA will normally be present in all or substantially all of the cells of the plant tissue after transformation and regeneration.

Agrobacterium tumefaciens and A. rhizogenes are plant-pathogenic soil bacteria, which genetically transform plant cells. The Ti- and Ri- plasmids of A. tumefaciens and A. rhizogenes, respectively, carry genes responsible for genetic transformation of the plant (Kado 1991). Vectors are based on the Agrobacterium Ti- or Ri-plasmid and utilize a natural system of DNA transfer into the plant genome. As part of this highly developed parasitism Agrobacterium transfers a defined part of its genomic information (the T-DNA; flanked by about 25 bp repeats, named left and right border) into the chromosomal DNA of the plant cell (Zupan 2000). By combined action of the so-called vir genes (part of the original Ti-plasmids) said DNA-transfer is mediated. For utilization of this natural system, Ti-plasmids were developed which lack the original tumor inducing genes ("disarmed vectors"). In a further improvement, the so-called "binary vector systems", the T-DNA was physically separated from the other functional elements of the Ti-plasmid (e.g., the vir genes), by being incorporated into a shuttle vector, which allowed easier handling (EP-A 120 516; US 4.940.838). These binary vectors comprise (beside the disarmed T-DNA with its border sequences), prokaryotic sequences for replication both in Agrobacterium and E. coli. It is an advantage of Agrobacterium-mediated transformation that in general only the DNA flanked by the borders is transferred into the genome and that preferentially only one copy is inserted. Descriptions of Agrobacterium vector systems and methods for Agrobacterium-mediated gene transfer are known in the art (Miki 1993; Gruber 1993; Moloney 1989).

Hence, for Agrobacterium-mediated transformation the genetic composition (e.g., comprising an expression cassette) is integrated into specific plasmids, either into a shuttle or intermediate vector, or into a binary vector. If a Ti or Ri plasmid is to be used for the transformation, at least the right border, but in most cases the right and left border, of the Ti or Ri plasmid T-DNA is linked to the expression cassette to be introduced in the form of a flanking region. Binary vectors are preferably used. Binary vectors are capable of replication both in E. coli and in Agrobacterium. They may comprise a selection marker gene and a linker or polylinker (for insertion of e.g. the expression cassette to be transferred) flanked by the right and left T-DNA border sequence. They can be transferred directly into Agrobacterium (Holsters 1978). The selection marker gene permits the selection of transformed Agrobacteria and is, for example, the nptIII gene, which confers resistance to kanamycin. The Agrobacterium which acts as host organism in this case should already contain a plasmid with the vir region. The latter is required for transferring the T-DNA to the plant cell. An Agrobacterium transformed in this way can be used for transforming plant cells. The use of T-DNA for transforming plant cells has been studied and described intensively (EP 120 516; Hoekema 1985; An et al. 1985).

Common binary vectors are based on "broad host range"-plasmids like pRK252 (Bevan 1984) or pTJS75 (Watson et al. 1985) derived from the P-type plasmid RK2. Most of these vectors are derivatives of pBIN19 (Bevan 1984). Various binary vectors are known, some of which are commercially available such as, for example, pB1101.2 or pBIN19 (Clontech Laboratories, Inc. USA). Additional vectors were improved with regard to size and handling (e.g. pPZP; Hajdukiewicz 1994). Improved vector systems are described also in WO 02/00900.

Various Agrobacterium strains can be successfully employed in the method of the invention. These include octopine strains, e.g., LBA4404 or agropine strains, e.g., EHA101 or EHA105. Suitable strains of A. tumefaciens for DNA transfer are for example EHA101pEHA101 (Hood et al. 1986), EHA105[pEHA105] (Li 1992), LBA4404[pAL4404] (Hoekema 1983), C58C1[pMP90] (Koncz & Schell 1986), and C58C1[pGV2260] (Deblaere 1985). Other suitable strains are Agrobacterium tumefaciens C58, a nopaline strain. Other suitable strains are A. tumefaciens C58C1 (Van Larebeke 1974), A136 (Watson 1975) or LBA4011 (Klapwijk 1980). The Agrobacterium strain may contain an octopine-type Ti-plasmid, preferably disarmed, such as pAL4404. Generally, when using octopine-type Ti-plasmids or helper plasmids, it is preferred that the virF gene be deleted or inactivated (Jarschow 1991). Additional suitable strains are C58C1[pGV2260] and C58C1 [pMP90]. Strain C58C1 [pGV2260] is an "Octopine-type" strain while C58C1 [pMP90] is a "Nopaline-type" strain. The genetic background of both is Agrobacterium strain C58. C58 is also the genetic background for strain GV3101 (the preferred strain for the transformation protocol of the invention).

The method of the invention can also be used in combination with particular Agrobacterium strains to further increase the transformation efficiency, such as Agrobacterium strains wherein the vir gene expression and/or induction thereof is altered due to the presence of mutant or chimeric virA or virG genes (e.g. Hansen 1994; Chen and Winans 1991; Scheeren-Groot et al., 1994). Possible are further combinations of Agrobacterium tumefaciens strain (e.g., LBA4404; Hiei 1994) with super-virulent plasmids (e.g., pTOK246-based vectors; Ishida 1996), so called super-virulent strains. A examples for super-virulent strains is the succinamopine strain EHA105. However, in another preferred embodiment of the invention, the employed Agrobacterium strain is not super-virulent. Is has been demonstrated that employment of these strains may - surprisingly - not lead to an improved but to a decreased transformation efficiency. The super-virulent strain EHA105 seems to damage petiole explants much more then "normal" strains reducing regeneration efficiency to only 16% of the explants. In comparison nearly twice the number of explants (31%) can be achieved using strain LBA4404 (see Example 4).

Most preferred the Agrobacterium strain employed during the method of the invention is a Nopaline strain (e.g., C58C1[pMP90] and GV3101) which demonstrated to be significantly superior with respect to overall regeneration / transformation efficiency than the octopine strain C58C1 [pGV2260].

A binary vector or any other vector can be modified by common DNA recombination techniques, multiplied in E. coli, and introduced into Agrobacterium by e.g., electroporation or other transformation techniques (Mozo 1991).

Agrobacteria are grown and used in a manner as known in the art. The vector comprising Agrobacterium strain may, for example, be grown for 3 days on YEB medium (see Example 2.6) supplemented with the appropriate antibiotic (e.g., 50 mg/l spectinomycin). Bacteria are collected with a loop from the solid medium and resuspended. In a preferred embodiment of the invention, Agrobacterium cultures are started by use of aliquots frozen at -80°C. For Agrobacterium treatment of isolated petioles, the bacteria are resuspended in the medium used for petiole culture.

The concentration of Agrobacterium used for infection and co-cultivation may need to be varied. Thus, a range of Agrobacterium concentrations from 10⁵ to 10¹⁰ cfu/ml and a range of co-cultivation periods from a few hours to 7 days can be used. The co-cultivation of Agrobacterium with the isolated petioles is in general carried out for 1 to 5, preferably 2 to 3 days.

It is possible, although not necessary, to employ one or more phenolic compounds in the medium prior to or during the Agrobacterium co-cultivation. "Plant phenolic compounds" or "plant phenolics" suitable within the scope of the invention are those isolated substituted phenolic molecules which are capable to induce a positive chemotactic response, particularly those who are capable to induce increased vir gene expression in a Ti-plasmid containing Agrobacterium sp., particularly a Ti-plasmid containing

Agrobacterium tumefaciens. Preferred is acetosyringone. Moreover, certain compounds, such as osmoprotectants (e.g. L-proline preferably at a concentration of about 700 mg/L or betaine), phytohormones (inter alia NAA), opines, or sugars, are expected to act synergistically when added in combination with plant phenolic compounds. The plant phenolic compound, particularly acetosyringone, can be added to the medium prior to contacting the isolated petioles with Agrobacteria (for e.g., several hours to one day). Possible concentrations of plant phenolic compounds in the medium range from about 25 µM to 700 µM. However, for the methods of the invention preferably no acetosyringone is employed. Particularly suited induction conditions for Agrobacterium tumefaciens have been described by Vernade et al. (1988). Efficiency of transformation with Agrobacterium can be enhanced by numerous other methods known in the art like for example vacuum infiltration (WO 00/58484), heat shock and/or centrifugation, addition of silver nitrate, sonication etc. However, the effects are rather small. Addition of silver nitrate for example seems to be without any visible positive effect.

Supplementation of the co-cultivation medium with antioxidants (e.g., dithiothreitol), or thiol compounds (e.g., L-cysteine, Olhoft et al. 2001) which can decrease tissue necrosis due to plant defense responses (like phenolic oxidation) may further improve the efficiency of Agrobacterium-mediated transformation.

However, in contrast to the methods disclosed in the art preferably no phenol-absorbing compound (like polyvinylpyrrolidone, Perl et al. 1996) is employed. PVP seems to have an disadvantageous effect on selection efficacy (presumably by unreliably binding antibiotic or herbicidal compounds). Therefore, in another preferred embodiment, the media employed during the transformation procedure do not comprise PVP (poly-vinylpyrrolidone).

It has been observed within this invention that transformation efficiency of the petioles by Agrobacterium can be significantly improved by keeping the pH of the co-cultivation medium in a range from 4.5 to 6.4, preferably 4.8 to 6.2, especially preferably 5.0 to 6.0. In an improved embodiment of the invention stabilization of the pH in this range is mediated by a combination of MES and potassium hydrogenphosphate buffers. Co-cultivation is carried out at pH 5.2 for three days.

After the co-cultivation with the bacteria described above, the explants are typically transferred to a selection and regeneration medium (which preferably contains B5 salts and vitamins, 3% sucrose).

The media as employed during the method of the invention may be optionally further supplemented with one or more plant growth regulator, like e.g., cytokinin compounds (e.g., 6-benzylaminopurine) and/or auxin compounds (e.g., 2,4-D). The term "plant growth regulator" (PGR) as used herein means naturally occurring or synthetic (not naturally occurring) compounds that can regulate plant growth and development. PGRs may act singly or in consort with one another or with other compounds (e.g., sugars, amino acids). The term "auxin" or "auxin compounds" comprises compounds which stimulate cellular elongation and division, differentiation of vascular tissue, fruit development, formation of adventitious roots, production of ethylene, and - in high concentrations - induce dedifferentiation (callus formation). The most common naturally occurring auxin is indoleacetic acid (IAA), which is transported polarly in roots and stems. Synthetic auxins are used extensively in modern agriculture. Auxin compounds comprise indole-3-butyric acid (IBA), naphthylacetic acid (NAA), and 2,4-dichlorphenoxyacetic acid (2,4-D), wherein 2,4-D is a more active compound.

The term "cytokinin" or "cytokinin compound" comprises compounds which stimulate cellular division, expansion of cotyledons, and growth of lateral buds. They delay senescence of detached leaves and, in combination with auxines (e.g. IAA), may influence formation of roots and shoots. Cytokinin compounds comprise, for example, 6-isopentenyladenine (IPA) and 6-benzyladenine/6-benzylaminopurine (BAP).

In contrast to the method disclosed in EP 270 615 cytokinins are included during the regeneration phase which in the present case enhance regeneration frequency (while in EP 270 615 it is stated that they decrease regeneration capacity). Preferably, no callus inducing auxin like e.g. 2,4-D is included during said phase (but only later during the root transformation phase in combination with cytokinins). It should be noted, that the method of the invention is not leading to callus induction and somatic embryogenesis but is based on induced organogenesis (shoot induction) from transformed cotyledonary petioles.

In another preferred embodiment, the media employed during co-cultivation (c) and/or selection and regeneration (d and e) comprises between about 1 mg/l and about 10 mg/l 6-benzylaminopurine (BA), preferably between 2 mg/l and 5 mg/l BAP, more preferably between 3 mg/l and 4 mg/l BAP.

It is furthermore especially preferred, that the media employed during co-cultivation (c) and/or selection and regeneration (d and e) comprise between about 0.1 mg/l and about 2 mg/l Gibberellic acid (GA3), preferably between 0.2 mg/l and 1 mg/l GA3, more preferably between 0.4 and 0.6 mg/l GA3.

In another preferred embodiment of the invention, the media employed during shoot elongation (f) comprise between about 0.001 mg/l and about 0.1 mg/l 6-benzylaminopurine (BAP), preferably between 0,002 mg/l and 0,005 mg/l BAP, and between about 0.01 mg/l and about 1 mg/l indole butyric acid (IBA), preferably between 0.05 mg/l and 0.5 mg/l IBA.

In another preferred embodiment of the invention, the media employed during rooting (g) comprise between about 0.01 mg/l and about 1 mg/l indole butyric acid (IBA), preferably between 0.05 mg/l and 0.5 mg/l IBA.

The selection and regeneration forming medium contains a bacteriocide (antibiotic), e.g., carbenicillin (500 mg/L) or Timentin™ (GlaxoSmithKline; a mixture of ticarcillin disodium and clavulanate potassium; 0.8 g Timentin contains 50 mg clavulanic acid with 750 mg ticarcillin. Chemically, ticarcillin disodium is N -(2-Carboxy-3,3-dimethyl-7-oxo-4-thia-1-azabicyclo[3.2.0]hept-6-yl)-3-thio-phenemalonamic acid disodium salt. Chemically, clavulanate potassium is potassium (Z)-(2R, 5R)-S-(2-hydroxyethylidene)-7-oxo-4-oxa-1-azabicyclo[3.2.0] heptane-2-carboxylate) and at least one compound, which in combination with the selectable marker gene of (a) allows for identification and/or selection of plant cells (e.g., a selective agent) may be applied.

Insertion of a selectable and/or screenable marker gene is comprised within the scope of the method of the invention. This may be advantageous e.g., for later use as a herbicide-resistance trait. The medium employed for culturing the transformed petioles or petioles derived shoots may further contain a compound used to select petioles or petiole derived shoots or plantlets containing a selectable or screenable DNA sequence. The compound used for said selection or screening can be any of a variety of well known selection compounds, such as antibiotics and herbicides. Preferred compounds can include geneticin (G-418) (aminoglycoside) (Nehra et al. 1994), glyphosate (Della-Cioppa et al. 1987) and bialaphos (Vasil et al. 1992; Weeks et al. 1993). The availability of alternative selection agents is an important requirement for commercial application of agriculture biotechnology. The use of kanamycin has been demonstrated to be highly efficient and is especially preferred. Further preferred selectable and screenable marker genes are disclosed below.

For example, with the kanamycin resistance gene (neomycin phosphotransferase, NPTII) as the selective marker, kanamycin at a concentration of from about 3 to 200 mg/l may be included in the medium. Typical concentrations for selection are 5 to 50 mg/l. The tissue is grown upon this medium for a period of 1 to 3 weeks, preferably about 7 days until shoots have developed from the cotyledonary petioles. Shoot formation begins in about 3-6 weeks depending on treatment and co-cultivation conditions.

After this time the newly developed shoots are isolated from the petioles and transferred to fresh selection and regeneration medium.

It has been found that use of a low-carbon-source medium during the regeneration procedure following co-cultivation of the Brassica cells with the transforming bacteria results in enhanced recovery and regeneration. Use of a low-carbon-source medium is believed to operate by forcing the cultured tissues to become dependent on other sources of energy, possibly photosynthesis. A low-carbon-source medium is one that contains no more than 2% by weight sucrose or the equivalent of a 2% by weight sucrose solution in caloric value. Other carbon sources (e.g., mono- or disaccharides) may give a similar effect provided that they provide the same caloric value.

The method of the invention is distinguished from the methods described in the art by at least the following features:
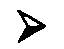 The use of Oxoid™ agar instead of Phytoagar™ (as used in the prior art) in certain concentrations resulted in an significantly improved recovery and transformation rate.
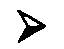 De Block (1989) is using hypocotyl target tissue instead of cotyledonary petioles.
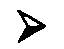 A combination of BAP with GA3 is used during co-cultivation, selection and regeneration, which is not used neither in EP 270 615 nor in Moloney (1989).

De Block (1989) is using PVP (polyvinylpyrrolidone) in the regeneration and selection step. This has proven to be of disadvantage and seems to stimulate unwanted Agrobacteria growth (presumably by binding antibiotics).

Transformed plant material (e.g., cells, tissues or plantlets) which express such marker genes are capable of developing in the presence of concentrations of a corresponding selection compound (e.g., antibiotic or herbicide) which suppresses growth of an untransformed wild type tissue. The resulting plants can be bred and hybridized in the customary fashion. Two or more generations should be grown in order to ensure that the genomic integration is stable and hereditary.

Other important aspects of the invention include the progeny of the transgenic plants prepared by the disclosed methods, as well as the cells derived from such progeny, and the seeds obtained from such progeny.

### CONSTITUTION OF THE T-DNA OF THE INVENTION

Preferably, the T-DNA inserted into the genome of the target Brassica plant comprises at least one expression cassette, which may - for example - facilitate expression of selection marker gene, trait genes, antisense RNA or double-stranded RNA. Preferably said expression cassettes comprise a promoter sequence functional in plant cells operatively linked to a nucleic acid sequence which - upon expression - confers an advantageous phenotype to the so transformed plant. The person skilled in the art is aware of numerous sequences which may be utilized in this context, e.g. to increase quality of food and feed, to produce chemicals, fine chemicals or pharmaceuticals (e.g., vitamins, oils, carbohydrates; Dunwell 2000), conferring resistance to herbicides, or conferring male sterility. Furthermore, growth, yield, and resistance against abiotic and biotic stress factors (like e.g., fungi, viruses or insects) may be enhanced. Advantageous properties may be conferred either by overexpressing proteins or by decreasing expression of endogenous proteins by e.g., expressing a corresponding antisense (Sheehy 1988; US 4,801,340; Mol 1990) or double-stranded RNA (Matzke 2000; Fire 1998; Waterhouse 1998; WO 99/32619; WO 99/53050; WO 00/68374; WO 00/44914; WO 00/44895; WO 00/49035; WO 00/63364).

For expression in plants, plant-specific promoters are preferred. The term "plant-specific promoter" is understood as meaning, in principle, any promoter which is capable of governing the expression of genes, in particular foreign genes, in plants or plant parts, plant cells, plant tissues or plant cultures. In this context, expression can be, for example, constitutive, inducible or development-dependent. The following are preferred:

### a) Constitutive promoters

"Constitutive" promoters refers to those promoters which ensure expression in a large number of, preferably all, tissues over a substantial period of plant development, preferably at all times during plant development. A plant promoter or promoter originating from a plant virus is especially preferably used. The promoter of the CaMV (cauliflower mosaic virus) 35S transcript (Franck 1980; Odell 1985; Shewmaker 1985; Gardner 1986) or the 19S CaMV promoter (US 5,352,605; WO 84/02913; Benfey 1989) are especially preferred. Another suitable constitutive promoter is the rice actin promoter (McElroy 1990), Rubisco small subunit (SSU) promoter (US 4,962,028), the legumin B promoter (GenBank Acc. No. X03677), the promoter of the nopaline synthase from Agrobacterium, the TR dual promoter, the OCS (octopine synthase) promoter from Agrobacterium, the ubiquitin promoter (Holtorf et al. 1995), the ubiquitin 1 promoter (Christensen et al. 1989, 1992; Bruce et al. 1989), the Smas promoter, the cinnamyl alcohol dehydrogenase promoter (US 5,683,439), the promoters of the vacuolar AT-Pase subunits, the pEMU promoter (Last et al. 1991); the MAS promoter (Velten et al. 1984) and maize H3 histone promoter (Lepetit et al. 1992; Atanassova et al. 1992), the promoter of the Arabidopsis thaliana nitrilase-1 gene (GenBank Acc. No.: U38846, nucleotides 3862 to 5325 or else 5342) or the promoter of a proline-rich protein from wheat (WO 91/13991), and further promoters of genes whose constitutive expression in plants.

### b) Tissue-specific or tissue-preferred promoters

Furthermore preferred are promoters with specificities for seeds, such as, for example, the phaseolin promoter (US 5,504,200; Bustos et al. 1989, Murai et al.1983; Sengupta-Gopalan et al. 1985), the promoter of the 2S albumin gene (Joseffson et al. 1987), the legumine promoter (Shirsat 1989), the USP (unknown seed protein) promoter (Bäumlein 1991a), the napin gene promoter (US 5,608,152; Stalberg et al. 1996), the promoter of the sucrose binding proteins (WO 00/26388) or the legumin B4 promoter (LeB4; Bäumlein et al. 1991 b, 1992), the Arabidopsis oleosin promoter (WO 98/45461), and the Brassica Bce4 promoter (WO 91/13980). Further preferred are a leaf-specific and light-induced promoter such as that from cab or Rubisco (Simpson 1985; Timko 1985); an anther-specific promoter such as that from LAT52 (Twell et al. 1989b); a pollen-specific promoter such as that from Zml3 (Guerrero et al. 1993); and a microspore-preferred promoter such as that from apg (Twell et al. 1993).

### c) Chemically inducible promoters

The expression cassettes may also contain a chemically inducible promoter (review article: Gatz et al. 1997), by means of which the expression of the exogenous gene in the plant can be controlled at a particular point in time. Such promoters such as, for example, the PRP1 promoter (Ward et al. 1993), a salicylic acid-inducible promoter (WO 95/19443), a benzenesulfonamide-inducible promoter (EP 0 388 186), a tetracyclin-inducible promoter (Gatz et al. 1991, 1992), an abscisic acid-inducible promoter EP 0 335 528) or an ethanol-cyclohexanone-inducible promoter (WO 93/21334) can likewise be used. Also suitable is the promoter of the glutathione-S transferase isoform II gene (GST-II-27), which can be activated by exogenously applied safeners such as, for example, N,N-diallyl-2,2-dichloroacetamide (W0 93/01294) and which is operable in a large number of tissues of both monocots and dicots. Further exemplary inducible promoters that can be utilized in the instant invention include that from the ACE1 system which responds to copper (Mett et al. 1993); or the In2 promoter from maize which responds to benzenesulfonamide herbicide safeners (Hershey et al. 1991; Gatz et al. 1994). A promoter that responds to an inducing agent to which plants do not normally respond can be utilized. An exemplary inducible promoter is the inducible promoter from a steroid hormone gene, the transcriptional activity of which is induced by a glucocorticosteroid hormone (Schena et al. 1991).

Particularly preferred are constitutive promoters. Furthermore, further promoters may be linked operably to the nucleic acid sequence to be expressed, which promoters make possible the expression in further plant tissues or in other organisms, such as, for example, E. coli bacteria. Suitable plant promoters are, in principle, all of the above-described promoters.

The genetic component and/or the expression cassette may comprise further genetic control sequences in addition to a promoter. The term "genetic control sequences" is to be understood in the broad sense and refers to all those sequences which have an effect on the materialization or the function of the expression cassette according to the invention. For example, genetic control sequences modify the transcription and translation in prokaryotic or eukaryotic organisms. Preferably, the expression cassettes according to the invention encompass a promoter functional in plants 5'-upstream of the nucleic acid sequence in question to be expressed recombinantly, and 3'-downstream a terminator sequence as additional genetic control sequence and, if appropriate, further customary regulatory elements, in each case linked operably to the nucleic acid sequence to be expressed recombinantly.

Genetic control sequences furthermore also encompass the 5'-untranslated regions, introns or noncoding 3'-region of genes, such as, for example, the actin-1 intron, or the Adh1-S introns 1, 2 and 6 (general reference: The Maize Handbook, Chapter 116, Freeling and Walbot, Eds., Springer, New York (1994)). It has been demonstrated that they may play a significant role in the regulation of gene expression. Thus, it has been demonstrated that 5'-untranslated sequences can enhance the transient expression of heterologous genes. Examples of translation enhancers which may be mentioned are the tobacco mosaic virus 5'-leader sequence (Gallie et al. 1987) and the like. Furthermore, they may promote tissue specificity (Rouster et al. 1998).

The expression cassette may advantageously comprise one or more enhancer sequences, linked operably to the promoter, which make possible an increased recombinant expression of the nucleic acid sequence. Additional advantageous sequences, such as further regulatory elements or terminators, may also be inserted at the 3'-end of the nucleic acid sequences to be expressed recombinantly. Polyadenylation signals which are suitable as control sequences are plant polyadenylation signals, preferably those which essentially correspond to T-DNA polyadenylation signals from Agrobacterium tumefaciens, in particular the OCS (octopine synthase) terminator and the NOS (nopaline synthase) terminator.

Control sequences are furthermore to be understood as those permitting removal of the inserted sequences from the genome. Methods based on the cre/lox (Sauer B 1998; Odell 1990; Dale 1991), FLP/FRT (Lysnik 1993), or Ac/Ds system (Wader et al. 1987; US 5,225,341; Baker et al. 1987; Lawson et al. 1994) permit an - if appropriate tissue-specific and/or inducible - removal of a specific DNA sequence from the genome of the host organism. Control sequences may in this context mean the specific flanking sequences (e.g., lox sequences), which later allow removal (e.g., by means of cre recombinase).

The genetic component and/or expression cassette of the invention may comprise further functional elements. The term functional element is to be understood in the broad sense and refers to all those elements which have an effect on the generation, amplification or function of the genetic component, expression cassettes or recombinant organisms according to the invention. Functional elements may include for example (but shall not be limited to):
1. Selectable Marker Genes
   Selectable marker genes are useful to select and separate successfully transformed or homologous recombined cells. Preferably, within the method of the invention one marker may be employed for selection in a prokaryotic host, while another marker may be employed for selection in a eukaryotic host, particularly the plant species host. The markers may be protection against a biocide, such as antibiotics, toxins, heavy metals, or the like, or may function by complementation, imparting prototrophy to an auxotrophic host. Preferred selectable marker genes for plants may include but are not be limited to the following:
   1.1 Positive selection markers
      Selection markers confer a resistance to a biocidal compound such as a metabolic inhibitor (e.g., 2-deoxyglucose-6-phosphate, WO 98/45456), antibiotics (e.g., kanamycin, G 418, bleomycin or hygromycin) or herbicides (e.g., phosphinothricin or glyphosate). Especially preferred selection markers are those which confer resistance to herbicides. Examples which may be mentioned are:
      - Phosphinothricin acetyltransferases (PAT; also named Bialophos® resistance; bar; De Block 1987; EP 0 333 033; US 4,975,374)
      - 5-enolpyruvylshikimate-3-phosphate synthase (EPSPS) conferring resistance to Glyphosate® (N-(phosphonomethyl)glycine) (Shah 1986)
      - Glyphosate® degrading enzymes (Glyphosate® oxidoreductase; gox),
      - Dalapon® inactivating dehalogenases (deh)
      - Sulfonylurea- and imidazolinone-inactivating acetolactate synthases (for example mutated ALS variants with, for example, the S4 and/or Hra mutation
      - Bromoxynil® degrading nitrilases (bxn)
      - Kanamycin- or. G418- resistance genes (NPTII; NPTI) coding e.g., for neomycin phosphotransferases (Fraley 1983)
      - 2-Deoxyglucose-6-phosphate phosphatase (DOG^{R}1-Gene product; WO 98/45456; EP 0 807 836) conferring resistance against 2-desoxyglucose (Randez-Gil 1995)
      - Hygromycin phosphotransferase (HPT), which mediates resistance to hygromycin (Vanden Elzen 1985).
      - Dihydrofolate reductase (Eichholtz 1987)

      Additional positive selectable marker genes of bacterial origin that confer resistance to antibiotics include the aadA gene, which confers resistance to the antibiotic spectinomycin, gentamycin acetyl transferase, streptomycin phosphotransferase (SPT), aminoglycoside-3-adenyl transferase and the bleomycin resistance determinant (Hayford 1988; Jones 1987; Svab 1990; Hille 1986).
      Genes like isopentenyltransferase from Agrobacterium tumefaciens (strain:PO22; Genbank Acc.-No.: AB025109) may - as a key enzyme of the cytokinin biosynthesis - facilitate regeneration of transformed plants (e.g., by selection on cytokinin-free medium). Corresponding selection methods are described (Ebinuma H et al. (2000) Proc Natl Acad Sci USA 94:2117-2121; Ebinuma H et al. (2000) Selection of Marker-free transgenic plants using the oncogenes (*ipt, rol* A, B, C) of Agrobacterium as selectable markers, In Molecular Biology of Woody Plants. Kluwer Academic Publishers). Additional positive selection markers, which confer a growth advantage to a transformed plant in comparison with a non-transformed one, are described e.g., in EP-A 0 601 092. Growth stimulation selection markers may include (but shall not be limited to) β-Glucuronidase (in combination with e.g., a cytokinin glucuronide), mannose-6-phosphate isomerase (in combination with mannose), UDP-galactose-4-epimerase (in combination with e.g., galactose), wherein mannose-6-phosphate isomerase in combination with mannose is especially preferred.
   1.2) Negative selection marker
      Negative selection markers are especially suitable to select organisms with defined deleted sequences comprising said marker (Koprek et al. 1999). Examples for negative selection marker comprise thymidine kinases (TK), cytosine deaminases (Gleave et al. 1999; Perera et al. 1993; Stougaard 1993), cytochrom P450 proteins (Koprek et al. 1999), haloalkan dehalogenases (Naested 1999), iaaH gene products (Sundaresan et al. 1995), cytosine deaminase codA (Schlaman & Hooykaas 1997), or tms2 gene products (Fedoroff & Smith 1993).
2) Reporter genes
   Reporter genes encode readily quantifiable proteins and, via their color or enzyme activity, make possible an assessment of the transformation efficacy, the site of expression or the time of expression. Very especially preferred in this context are genes encoding reporter proteins (Schenborn 1999) such as the green fluorescent protein (GFP) (Sheen 1995; Haseloff 1997; Reichel 1996; Tian 1997; WO 97/41228; Chui 1996; Leffel 1997), chloramphenicol transferase, a luciferase (Ow 1986; Millar 1992), the aequorin gene (Prasher 1985), β-galactosidase, R locus gene (encoding a protein which regulates the production of anthocyanin pigments (red coloring) in plant tissue and thus makes possible the direct analysis of the promoter activity without addition of further auxiliary substances or chromogenic substrates (Dellaporta 1988; Ludwig 1990), with β-gtucuronidase (GUS) being very especially preferred (Jefferson 1987a,b). β-glucuronidase (GUS) expression is detected by a blue color on incubation of the tissue with 5-bromo-4-chloro-3-indolyl-β-D-glucuronic acid, bacterial luciferase (LUX) expression is detected by light emission; firefly luciferase (LUC) expression is detected by light emission after incubation with luciferin; and galactosidase expression is detected by a bright blue color after the tissue was stained with 5-bromo-4-chloro-3-indolyl-β-D-galactopyranoside. Reporter genes may also be used as scorable markers as alternatives to antibiotic resistance markers. Such markers are used to detect the presence or to measure the level of expression of the transferred gene. The use of scorable markers in plants to identify or tag genetically modified cells works well only when efficiency of modification of the cell is high.
3) Origins of replication, which ensure amplification of the expression cassettes or vectors according to the invention in, for example, E. coli. Examples which may be mentioned are ORI (origin of DNA replication), the pBR322 ori or the P15A ori (Maniatis 1989). Additional examples for replication systems functional in E. coli, are ColE1, pSC101, pACYC184, or the like. In addition to or in place of the E. coli replication system, a broad host range replication system may be employed, such as the replication systems of the P-1 Incompatibility plasmids; e.g., pRK290. These plasmids are particularly effective with armed and disarmed Ti-plasmids for transfer of T-DNA to the plant species host.
4) Elements which are necessary for Agrobacterium-mediated plant transformation, such as, for example, the right and/or - optionally - left border of the T-DNA or the vir region.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the composition, methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims. All publications, patents, and patent applications cited herein are hereby incorporated by reference for all purposes.

### Examples:

### General methods

Unless otherwise specified, all chemicals were from Fluka (Buchs, Switzerland), Merck (Darmstadt, Germany), Roth (Karlsruhe, Germany), Serva (Heidelberg, Germany) and Sigma (Deisenhofen, Germany). Restriction enzymes, DNA-modifying enzymes and molecular biological kits were from Amersham-Pharmacia (Freiburg, Germany), Biometra (Göttingen, Germany), Roche (Mannheim, Germany), New England Biolabs (Schwalbach, Germany), Novagen (Madison, Wisconsin, USA), Perkin-Elmer (Weiterstadt, Germany), Qiagen (Hilden, Germany), Stratagene (Amsterdam, Netherlands), Invitrogen (Karlsruhe, Germany) and Ambion (Cambridgeshire, UK). The reagents used were employed in accordance with the manufacturer's instructions. The following examples are offered by way of illustration and not by way of limitation.

### Example 1: Brassica transformation standard protocol

### 1.1 Components of media used during the process (details s. Appendix I):

| | **Basal/ pH** | **Sucrose** (%) | **Agar**^{**4**} (%) | **BAP** mg/l | **MES** mg/l | **GA3** mg/l | **IBA** mg/l | **Tim.**^{**5**} mg/l | **myol**^{**6**} mg/l | **AS**^{**7**} mg/l | **Kan**^{**8**} mg/l |
|---|---|---|---|---|---|---|---|---|---|---|---|
| **Germination** | 1xMSB5¹ pH 5.8 | 3 | 0.8 | - | - | - | - | - | - | | - |
| **Co-cultivation** | 1xMSB5¹ pH 5.2 | 3 | 0.8 | 3.75 | 500 | 0.5 | - | - | - | | - |
| **Regeneration / Selection** | 1xMSB5¹ pH 5.2 | 3 | 0.8 | 3.75 | 500 | 0.5 | - | 300 | - | | 18 |
| **Shoot elongation / Rooting A6** | 1xMSV² pH 5.8 | 2 | 0.5 | 0.0025 | 500 | - | 0.1 | 150 | 100 | 40 | 15 |
| **Rooting A7**^{**3**} | 1xMSV² pH 5.8 | 2 | 0.5 | - | 500 | - | 0.1 | 150 | 100 | 40 | 15 |

| | | | | | | | | | | | |
|---|---|---|---|---|---|---|---|---|---|---|---|
| ¹ MSB5: MS medium incl. Gamborg B5 vitamins | | | | | | | | | | | |
| ² MSV: MS medium incl. MS salts and vitamins | | | | | | | | | | | |
| ³ In several cases root formation already occurs on A6 medium and transfer to A7 can be omitted | | | | | | | | | | | |
| ⁴ Oxoid™ agar; | | | | | | | | | | | |
| ⁵ Timentin™; | | | | | | | | | | | |
| ⁶myo Inositol; | | | | | | | | | | | |
| ⁷ Adeninsulfate; | | | | | | | | | | | |
| ⁸Kanamycin | | | | | | | | | | | |

### 1.2 Comparison with media / conditions disclosed in the prior art:

| | Method of the invention | EP 270 615 | Moloney et al. 1989 | De Block et al. 1989 |
|---|---|---|---|---|
| Target Tissue | Cotyledonary petioles | 1: Leaf disks 2: Hypocotyl explants | Cotyledonary petioles | Hypocotyl explants |
| Medium Solidifier | Oxoid ™agar | Pytagar™ | Pytagar* | agar / agarose |
| Germination | MSB5, 0.8% agar 3% sucrose | 1: soil 2:1/10 MS medium | MS, 0.7% agar, 2% sucrose | MS, 1/2 salts, no vitamins, 0.8% agar, 2% sucrose |
| Co-cultivation | MSB5, 0.8% agar 3% sucrose 3.75 mg/l BAP 0.5 mg/l GA3 500 mg/l MES | MSB5, 0.6% agar 3% sucrose 1 mg/l 2,4-D 1 mg/l kinetin | MS, 0.7% agar, 3% sucrose 4.5mg/l (20µM) BAP | MS 500 mg/l MES 0.1 mg/l NAA 1 mg/l BAP 0.01 mg/l GA3 |
| Regeneration / Selection | MSB5, 0.8% agar 3% sucrose 3.75 mg/l BAP 0.5 mg/l GA3 500 mg/l MES 300 mg/l Timentin™ 18 mg/l kanamycin | MSB5, 0.6% agar 3% sucrose 1 mg/l 2,4-D 1 mg/l kinetin 0.5 g/l carbenicillin 10-50 mg/l kanamycin | MS, 0.7% agar, 3% sucrose 4.5 mg/l (20µM) BAP 0.5 g/l carbenicillin 15 mg/l kanamycin | MS 500 mg/l MES 40 mg/l Adenin-SO4 0.5 mg/l PVP 2 % sucrose 0.5 % agarose 0.1 mg/l NAA 1 mg/l BAP 0.01 mg/l GA3 2-10mg/l AgNO3 0.5 g/l carbenicillin 50 mg/l kanamycin |
| Further Regeneration | '''' | MSB5, 0.6% agar 3% sucrose 3 mg/l BAP 1 mg/l zeatin 0.5 g/l carbenicillin 10-50 mg/l kanamycin | '''' | MS 500 mg/l MES 40 mg/l adenin-SO4 0.5 mg/l PVP 2 % sucrose 0.5 % agarose 0.0025mg/l BAP 0.25 g/l carbenicillin |
| Shoot Elongation | MS 500 mg/l MES 40 mg/l adenine-SO4 100 mg/l myo-inositol 2 % sucrose 0.5 % agarose 0.0025 mg/l BAP 0.1 mg/l IBA 150 mg/l Timentin™15 mg/l Kanamycin | MSB5.0.6% agar 3% sucrose 0.3 g/l carbenicillin | MS, 0.7% agar, 3% sucrose 0.5 g/l carbenicillin 15 mg/l kanamycin | MS, 1/2 salts, no Vitamins, 0.5% agar, 1 % sucrose 0.0025mg/l BAP 0.1 g/l carbenicillin |
| Rooting | MS 500 mg/l MES 40 mg/l adenine-SO4 100 mg/l myo-Inosit 2 % sucrose 0.5 % agarose 0.1 mg/l IBA 150 mg/l Timentin™ 15 mg/l Kanamycin | MSB5.0.6% agar 1% sucrose 2 mg/IBA 0.2 g/l Carbenicillin | MS, 0.7% agar, 3% sucrose 2 mg/l IBA BAP 0.5 g/l Carbenicillin 15 mg/l Kanamycin | MS, 1/2 salts, no vitamins, 1% sucrose 0.1 mg/l IBA 0.1 g/l Carbenicillin |

The method of the invention is distinguished from the methods described in the art by at least the following features:
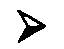 3% sucrose is used in the germination step instead of 2% disclosed in the art. The resulting seedlings are smaller but significantly more regenerable. The reduction of sucrose in the shoot elongation / rooting step seems to stimulate shoot elongation.
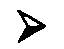 De Block (1989) is using hypocotyl target tissue instead of cotyledonary petioles.
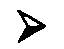 A combination of BAP with GA3 is used during cocultivation, selection and regeneration, which is not used neither in EP 270 615) nor in Moloney (1989)
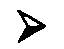 DeBlock (1989) is using PVP (polyvinylpyrrolidone) in the regeneration and selection step. This has proven to be of disadvantage and seems to stimulate unwanted Agrobacterium growth (presumably by binding antibiotics).
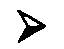 The use of Oxoid agar instead of Phytoagar™ resulted in an significantly improved recovery and transformation rate.

### 2. Standard Protocol

### 2.1. Stock Solutions (Components from Duchefa; Germany)

| **Compound** | Cat.-No. | Conc. of Stock. (mg/ml) | Solvent / Mode of Production | Storage | | Use for |
|---|---|---|---|---|---|---|
| | | | | "Long term" | "Short term" | |
| **Kanamycine** | K0126 | 10 | H₂O dest., filter-sterilized | -20°C | -20°C | Co-Cultivation Selection, A6-medium - |
| **Timentln** | T0190 | 300 | H₂O dest., filter-sterilized | -20°C | -20°C | Selection, A6-medium |
| **BAP** | B0904 | 1 | solve 100 mg in 2 ml 1 M NaOH, add H₂O dest. to 100 ml | 4°C | 4°C | Co-Cultur, Selection, A6-medium |
| **GA3** | G0907 | 1 | solve 100 mg in 10 ml ethanol, add H₂O dest. to 100 ml | 4°C | 4°C | Selection |
| **IBA (lightsensitive)** | I0902 | 1 | solve 100 mg in 10 ml ethanol, add H₂O dest. to 100 ml, adjust pH to 5.8, filter-sterilized | -20°C | 4°C, in the dark | A6-medium |

| (Components from Sigma, Germany) | | | | | |
|---|---|---|---|---|---|
| **Compound** | Cat.-No. | Conc. of Stock (mg/ml) | Solvent / Mode of Production | Storage | Use for |
| **Kanamycin** | K1377 | 10 | H₂O dest., filter-sterilized | -20°C | A6-medium |
| **BAP** | B3408 | 1 | solve 100 mg in 2 ml 1 M NaOH, add H₂O dest. to 100 ml | 4°C | A6-medium |

### 2.2 Addtional Media Components

| **Component** | **Producer** | **Cat.-No.** | **Use for** |
|---|---|---|---|
| Adenine sulfate | Sigma | A9126 | A6-medium, Shoot elongation, Rooting |
| Agar Bacteriologic | Oxoid, Nr 1 | LP0011T | All media excluding YEB |
| Beef extract | Difco (BD) | 211520 | YEB, |
| MES | Duchefa | M1503 | Co-Cultivation, Selection medium, A6 medium |
| MgSO₄ | Merck | 1.05886.0500 | YEB, |
| MS | Sigma | M5519 | A6 medium |
| MSB5 Medium | Duchefa | M0231.0050 | Co-cultivation Selection medium |
| Myo-Inositol | Duchefa | I0609 | A6-Medium Shoot elongation, Rooting |
| Pepton | Duchefa | P1328 | YEB, |
| Plant Agar | Duchefa | P1001 | YEB |
| Sucrose | Duchefa | S-0809 | Co-cultivation, YEB, selection |
| Sucrose | Sigma | S-1888 | A6 medium |
| Yeast extract | Duchefa | Y1333 | YEB, |

### 2.3. Cultivation vessels

| **Type** | **Producers Name** | **Producer** | **Cat.-No.** | **Use for** |
|---|---|---|---|---|
| Petri dish, small | Petri dish with ventilation ribs, 60, 0/15 mm | Greiner | 628102 | Dipping |
| Petri dish, | dish 94/16 | Greiner | 633180 | Co-cultivation |
| Petri dish, high board | Petri dish with cams, 100, 0/20 mm | Greiner | 664102 | Selective regeneration until shoot establishment |

For all sterile filtered media BAP and GA3 are added to the medium before adjusting the pH. Timentin, kanamycin, and IBA are added only just before pourring of the medium into the plates.

### 2.4. Sterilization of Brassica napus seeds

200 to 500 seeds incubated under stirring in 50 to 70 ml 70% ethanol in a 100 ml Erlenmeyer vessel for 2 min at room temperature. The ethanol is discarded and about 70 ml 55°C warm tub water is added and incubated on a heating plate under slight stirring for 15 min. The water is discarded and the seeds are transferred to 1,5% (w/w) sodium hypochlorite solution (300 ml Erlenmeyer vessel) and stirred 10 min at room temperature. The sodium hypochlorite solution is discarded and the seeds are washed 3 to 4 times (until the smell of sodium hypchlorite has vanished) with 250 ml sterile H₂O under shaking. For the last washing step the seeds are kept in the water for about 10 min. The water is discarded and the seeds are dried in the air on a sterile piece of filter paper.

### 2.5. Germination of Brassica napus seeds

MSB5-Medium: MSB5 (Murashige & Skoog medium comprising Gamborg B5 salts and vitamins; Gamborg et al. (1968) Exp Cell Research 50:151-158; Duchefa), 3% Sucrose (Duchefa; 30 g/l), 0,8% Oxoid™ agar (8 g/l), pH 5,8, autoclaved. (The prepared medium can be stored for a maximum of 14 days for use at room temperature)

The sterilized seeds are put on germination medium MSB5 (25 to 30 seeds for each germination vessel). Germination is for 4 days at 24°C under moderate illumination (< 50 µMol/m²s).

### 2.6. Growth of the Agrobacterium strain

YEB-Medium: Beef extract (BD) 5 g/l; Yeast extract (Duchefa, Netherlands) 1 g/l; Peptone (Duchefa) 5 g/l; Sucrose (Duchefa) 5 g/l; MgSO₄ (Merck, Germany) 0.49 g/l.

Starting form a Agrobacterium glycerol stock (1 ml, in 10% glycerol, stored at -80°C) 50 ml YEB (including appropriate antibiotic) are inoculated with the Agrobacterium strain comprising the binary plasmid to be transformed into the Brassica cells. The culture is incubated for about 24 h at 28°C under shaking.

### 2.7. Preparation of cotyledonary petioles

Co-cultivation medium (final concentration): MSB5 Ready medium (Duchefa) comprising 0.8% Oxoid™ Agar, 30 g/l sucrose (Duchefa), 3,75 mg/l BAP (Duchefa), 0,5 g/l MES (Duchefa), 0,5 mg/l GA3 (Duchefa), pH 5,2 (sterile filtered before addition of autoclaved agar component). The medium in poured into flat Petri dishes.

Petioles are isolated from germinated Brassica seeds are inserted into the co-cultivation medium in a way that the cotyledon does not get into contact with the agar.

### 2.8 Preparation of bacteria suspension and explant infection

The overnight culture (25 ml) is precipitated (6000 rpm, 8 min, 4°C) and the bacteria pellet resuspended in an equal volume of liquid co-cultivation medium MSB5 (comprising BAP and GA3) by slight shaking. The resulting suspension can be stored in a refrigerator (4 to 6°C) for up to 7 days. The optical density (OD600) is determined and adjusted with the same medium to 0.5.

Petioles are individually dipped for about 2-3 seconds into the bacteria suspension. The cotyledon should not get into contact with the suspension. Thereafter the explants are re-inserted into the agar plates for the three day co-cultivation period (24°C; moderate illumination 50 µMol/m²S).

### 2.9. Selective Regeneration

After co-cultivation the explants are inserted (10 explants / plate) upright into the following Regeneration / Selection Medium (final concentration): Medium Component: MSB5 Ready Medium (Duchefa) comprising 0.8% Oxoid™ agar, 30 g/l sucrose (Duchefa), 3,75 mg/l BAP (Duchefa), 0,5 g/l MES (Duchefa), 0,5 mg/l GA3 (Duchefa), 18 mg/l Kanamycin (Duchefa, 1,8 ml/l) and 300 mg/l Timentin™ (Duchefa, 1,0 ml/l) pH 5,2 (sterile filtered before addition of autoclaved agar component). The medium in poured into flat Petri dishes.

Explants are cultivated on said plates for about 4 weeks at 24°C. Thereafter they are transferred on fresh plates. As soon as explants have developed one or more reasonable developed shoots (not to small, at least 0.5 cm in heights) they are transferred on A6 Medium. As soon as elongations starts and the shoots have good developed shoot axis, they are separated from the explant and further cultivated separately on A7 Medium. Samples for determination of transgenic status are taken after roots have been developed.

### Medium A6 - Final Concentrations:

- 1 x MS - Salts and Vitamins (Sigma)
- 2% Sucrose (Sigma) 20 g/l
- myo-Inositol (Duchefa) 100 mg/l
- Adenine sulfate (Sigma) 40 mg/l
- MES (Duchefa) 500 mg/l
- BAP (Sigma) 0,0025 mg/l
- pH 5,8

The solution is prepared double concentrated and sterile filtered. A 0.5% Oxoid™ agar solution is prepared (5 g/l) in H₂O in double concentration and autoclaved. Sterile filtered medium components and liquefied agar components are mixed. After mixing, 150 mg/l Timentin™ (Duchefa), 15 mg/l kanamycin (Sigma) and 0,1 mg/l IBA are added. A7 medium is identical to A6 medium but comprises no BAP.

### 2.10. Transfer to soil/ greenhouse conditions

In vitro plants with good root development and proven transgenic status are taken from the agar medium, cleaned from residual agar, dipped into Rootone™ and planted into 6 cm pots with standard greenhouse substrate.

### Example 3: Influence of agar type on regeneration efficiency

Various agar types were compared for their influence on transformation efficiency. Phytoagar™ (Duchefa; Cat.-No.: P1003), which is the standard agar used in plant biotechnology, was compared with bacteriological agar (Oxoid™; Cat.-No.: LP0011T) (Tab. 7).

**Tab. 7:**

| Temporal course of regeneration in presence of different agar types | | | | | | |
|---|---|---|---|---|---|---|
| Weeks | **Phytoagar™** | | | **Oxoid™ agar** | | |
| | #Ex^{a} | Shoots | % | #Ex^{a} | Shoots | % |
| 0 | 607 | 0 | 0 | 545 | 0 | 0 |
| 4 | | 19 | 3,1 | | 41 | 7,5 |
| 8 | | 20 | 3,3 | | 37 | 6,8 |
| 12 | | 40 | 6,6 | | 50 | 9,2 |
| 16 | | 38 | 6,3 | | 47 | 8,6 |
| 20 | | 24 | 4,0 | | 50 | 9,2 |

The fluctuation within one experiment results from the fact, that under selective regeneration, new shoots are formed while others (non-transgenic) degenerate. However, the use of Oxoid™ agar resulted in a highly significant increase in regeneration efficiency in comparison to the standard Phytoagar™.Oxoid™ agar is distinguished from Phytoagar mainly by its low ion content (magnesium and calcium) and its low electroendosmotic value.

| Parameter | Oxoid™ Agar | Phytoagar™ |
|---|---|---|
| Ca²⁺ | 100 ppm | 3702 ppm |
| Mg²⁺ | 40 ppm | 1295 ppm |
| Electroendosmosis (Mr) | 0.32 | 0.52 |

### Example 4: Choice of Agrobacterium strain

Transformation was carried out as described in the standard protocol above (beside that for comparison reasons the regeneration medium did not comprise kanamycin). The choice of the Agrobacterium strain on regeneration was assessed. Cotyledonary petioles were dipped in a) medium without Agrobacteria b) medium comprising the octopine strain LBA4404, and c) medium comprising the super-virulent succinamopine strain EHA105, respectively.

**Tab. 1:**

| Influence of A. tumefaciens strains and culture medium of regeneration efficiency of cotyledonary petioles. | | | | | | | | | | |
|---|---|---|---|---|---|---|---|---|---|---|
| **Cultivar** | **Medium** | **No Co-Cultivation** | | | **Co-Cultivation with LBA4404** | | | **Co-Cultivation with EHA105** | | |
| | | Total^{c} | SFE^{d} | % | Total^{c} | SFE^{d} | % | Total^{c} | SFE^{d} | % |
| Westar | SP^{a} | 60 | 34 | 57 | 208 | 65 | 31 | 960 | 153 | 16 |
| | + KIN^{b} | 60 | 48 | 80 | 208 | 73 | 35 | n.t. | n.t. | n.t. |
| Drakkar | SP^{a} | 60 | 19 | 32 | 208 | 57 | 27 | n.t. | n.t. | n.t. |
| | + KIN^{b} | 60 | 39 | 65 | 208 | 87 | 42 | n.t. | n.t. | n.t. |

| | | | | | | | | | | |
|---|---|---|---|---|---|---|---|---|---|---|
| a - Standard protocol, | | | | | | | | | | |
| b - Addition of 2.0 mg/l kinetin to regeneration medium; | | | | | | | | | | |
| c - Total number of explants tested; | | | | | | | | | | |
| d - shoot forming explants; n.t. - not tested | | | | | | | | | | |

Regeneration efficiency of both Brassica napus cultivars increased significantly when treated with Agrobacteria. In this respect the super-virulent strain EHA105 is significantly damaging the explants reducing regeneration efficiency to only 16% of the explants. In comparison, nearly the double number of explants (31%) can be achieved using strain LBA4404. This demonstrates that surprisingly the use of a super-virulent strain is a disadvantage in transforming cotyledonary petioles of Brassica species.

Furthermore, it could be demonstrated, especially for the Drakkar cultivar, that supplementation with kinetin is significantly increasing regeneration efficiency (27 to 42%).

### Example 5: Comparison of octopine- and nopaline Agrobacterium strains

For a comparison the respective Ti-plasmids has to be introduced into the same genetic Agrobacterium background. This is the case for strains C58C1 [pGV2260] and C58C1 [pMP90]. Strain C58C1 [pGV2260] is a "octopine-type" strain while C58C1 [pMP90] is a "nopaline-type" strain. The genetic background of both is Agrobacterium strain C58. C58 is also the genetic background for strain GV3101 (the preferred strain for the transformation protocol of the invention).

Both strains are transformed with the construct UH127 (for details see WO 03/008596), a plasmid comprising the nptII selection marker under control of the Nos-P promoter and the reporter gene uidA-INT under control of the nitrilase I gene promoter (Nit-P). The uidA-INT gene product allows easy detection of the construct by histological staining for glucuronidase activity.

Both of the above mentioned strains were used for transformation based on the above specified standard protocol. In a second experiment, a modified A5 medium was employed comprising (De Block (1989), comprising MS salts, B5 vitamins, pH 5.8, 3% sucrose, 40 mg/l adenine sulfate, 1 mg/l BAP, 0.01 mg/l GA3, 5 mg/l AgNO₃. Both media were supplemented with 300 mg/l Timentin™, which suppresses growth of A. tumefaciens, and 18 mg/l kanamycin for selection of the transformants.

Four weeks after transformation petioles were cut and used for histological GUS staining. Explants demonstrating a typical blue GUS-staining were counted, in addition the number of GUS positive spots on each explant were counted (Tab. 2).

**Tab. 2:**

| Influence of A. tumefaciens strains and regeneration medium on regeneration efficiency. | | | | |
|---|---|---|---|---|
| **Strain** | **Medium** | **# Explants** | **# GUS positive** | **%** |
| C58C1[pGV2260] | MSB5 | 20 | 9 | 45 |
| C58C1[pMP90] | MSB5 | 20 | 9 | 45 |
| C58C1[pGV2260] | A5 | 21 | 11 | 52 |
| C58C1[pMP90] | A5 | 19 | 13 | 68 |

During this early step of transformation the modified A5 medium seems to be superior in comparison to the MSB5 medium of the standard protocol. While using the MSB5 medium both strain result in equivalent transformation efficiency, with the modified A5 medium a significantly higher efficiency for the nopaline strain could be observed.

The same experiment was assessed with respect to the transformation events of each explant (Tab. 3).

**Tab. 3:**

| Influence of A. tumefaciens strains and regeneration medium on number of transformation event for individual explants. | | | | |
|---|---|---|---|---|
| **Strain** | **Medium** | **# GUS-pos. explants** | **No of all Spots** | **Spots per explant** |
| C58C1[pGV2260] | MSB5 | 9 | 20 | 2.2 |
| C58C1[pMP90] | MSB5 | 9 | 24 | 2.7 |
| C58C1[pGV2260] | A5 | 11 | 23 | 2.1 |
| C58C1[pMP90] | A5 | 13 | 16 | 1.2 |

For the octopine strain, both media result in the same number of events (two in average). In contrast the number of events for the nopaline strains seems to be influenced by the choice of the culture medium. While explants which were incubated on MSB5 standard medium MSB5 demonstrate about 3 GUS-positive spots, the corresponding explants cultured on the modified A5 medium had only a low spot number of about one per explant. Based on this result, the MSB5 medium seems to result in the higher number of insertion events, while not tremendously reducing regeneration efficiency per se.

By additional control experiments, influence of the slightly different pH (pH 5.2 for the standard protocol; pH 5.8 in the modified A5 medium) was ruled out. Also an influence of AgNO₃, that was only used in the modified A5 medium, was ruled out. AS a consequence, differences in regeneration efficiency seem to be based on the different composition of growth regulators employed in the media.

### Example 6: Comparison of octopine- and nopaline-type strains for stable transformation

The results of Example 3 were based on an evaluation (mainly on cellular level) 4 weeks after transformation. This experiment is dedicated to assess the influence on the efficiency of regeneration of whole, transgenic plants. Under standard conditions strains C58C1 [pMP90], C58C1 [pGV2260] and GV3101 (nopaline strain), respectively, were employed for transformation. The binary vector construct was UH127 (or a similar construct differing only in the promoter in front of the uidA-INT gene) with a Nos-P-nptII selection marker cassette and a uidA-INT reporter gene construct. Plants selected for kanamycin resistance were sampled and analyzed by genomic PCR for stable insertion of the uidA-INT gene (Tab. 4). The nopaline strains C58C1[pMP90] and GV3101 demonstrated to be significantly superior with respect to overall regeneration / transformation efficiency then the octopine strain C58C1 [pGV2260].

**Tab. 4:**

| Comparison of octopine- and nopaline-type strains for stable transformation and molecular analysis of putative transgenic plants by genomic PCR | | | | | | |
|---|---|---|---|---|---|---|
| **Strain** | **Type** | **Binary vector** | **# Explants** | **PCR analysis** | | **%** |
| | | | | **# tested** | **uidA-INT positive** | |
| C58C1[pMP90] | Nopaline | UH127 nit-P-GUS | 120 | 18 | 14 | 12 |
| C58C1[pGV2260] | Octopine | UH127 nit-P-GUS | 120 | 10 | 3 | 2 |
| GV3101 | Nopaline | UH197 TPT-P-GUS | 150 | 27 | 13 | 9 |
| GV3101 | Nopaline | UH198 FNR-P-GUS | 150 | 41 | 19 | 13 |

### Example 7: Influence of GA₃ on regeneration efficiency

The influence of GA₃ on regeneration efficiency was assessed by culturing transformed explants after co-cultivation on regeneration medium with various GA₃ concentrations (all other regeneration conditions remained unchanged). Evaluation was done every 4 weeks (Tab. 5). It was demonstrated that up to 0.5 mg/l GA₃ stimulates regeneration, while 1 mg/l is already reducing efficiency.

**Tab. 5: :**

| Influence of GA₃ concentration on regeneration efficiency | | | | | | | | | | | | |
|---|---|---|---|---|---|---|---|---|---|---|---|---|
| Week | 0 GA₃ | | | 0.1 mg/l GA₃ | | | 0.5 mg/l GA₃ | | | 1 mg/l GA₃ | | |
| | #Ex^{a} | P^{b} | Shoots | #Ex^{a} | P^{b} | Shoots | #Ex^{a} | P^{b} | Shoots | #Ex^{a} | P^{b} | Shoots |
| | 220 | 0 | 0 | 250 | 0 | 0 | 220 | 0 | 0 | 220 | 0 | 0 |
| 4 | 200 | 0 | 0 | 208 | 0 | 0 | 100 | 0 | 0 | 112 | 0 | 0 |
| 8 | | 40 | 3 | | 45 | 9 | | 38 | 14 | | 0 | 2 |
| 12 | | 10 | 5 | | 20 | 10 | | 10 | 18 | | n.d. | 6 |
| 16 | | 2 | 7 | | 7 | 12 | | 8 | 20 | | n.d. | 6 |

| | | | | | | | | | | | | |
|---|---|---|---|---|---|---|---|---|---|---|---|---|
| a - Number of explants at the beginning of the experiment | | | | | | | | | | | | |
| b - Number of primordia (regenerated structures which generally develop into shoots) | | | | | | | | | | | | |

### Example 8: Influence of GA₃ during co-cultivation on transformation efficiency

Two lines of experiment were conducted:
1. Variation I is identical to the standard protocol but without addition of GA₃ during co-culture.
2. Variation II was supplemented with 0.5 mg/l GA3 during the co-culture. The A. tumefaciens strain C58C1[pMP90] comprising a binary vector with a GFP reporter gene construct was employed for transformation. 12 weeks after starting selective regeneration 22% of the explants of Variation I and 28% of Variation II had produced shoots. The GA3 supplementation to the co-cultivation medium therefore was found to result in a small, but significant increase in regeneration efficiency (as measured by the number of shoots per explants): While without GA₃ in the co-culture explants develop on average 1.9 shoots per explant, there were 2.4 per explant when GA₃ was employed also during co-cultivation.

The transgenic status of the regenerated shoots was assessed by genomic PCR (based on amplification of the nptII and virD2 gene) (Tab. 6).

**Tab. 6:**

| Influence of GA3 during co-cultivation on transformation efficiency | | | | | | |
|---|---|---|---|---|---|---|
| **Variant** | **Explants (#)** | **Analyzed Shoots (#)** | ***npt*****II + (#)** | ***VirD2* + (#)** | **Min (%)** ^{**a**} | **PTE (%)**^{**b**} |
| I | 338 | 44 | 18 | 5 | 30 | 13 |
| II | 1050 | 168 | 42 | 1 | 24 | 16 |

| | | | | | | |
|---|---|---|---|---|---|---|
| a - Percentage (%) of transgenic shoots of selected shoots (virD positive plants were considered non-transgenic and not counted) | | | | | | |
| b - Transformation efficiency: Percentage (%) of transgenic shoots of transformed explants. | | | | | | |

### Example 9: Preculture of Agrobacteria prior to transformation

Agrobacteria were cultivated in YEB liquid medium over night at 28°C, centrifuged as described above and resuspended to a final OD600 of 0.5. In comparison, bacteria were resuspended in a quantity of co-cultivation medium and stored at 5°C prior to use. Just before dipping, the OD600 was adjusted to 0.5 with the same medium. Viability of the bacteria in the culture was found be unchanged. Furthermore, no loss of Ti plasmids could be observed. Results are presented in Tab. 8.

**Tab. 8:**

| Influence of 7-day storage of a pre-culture of Agrobacteria at 5°C. The experiment was evaluated after 6 weeks. | | | | | | | |
|---|---|---|---|---|---|---|---|
| Experiment | Variant | Explant (#) | Still existing explants (#) | Shoot forming explants (#) | | Shoots / explant^{a} | |
| | | | | # | % | # | % |
| I | Standard | 380 | 120 | 86 | 23 | 6/6 | 2/2 |
| | Agro-Preculture | 400 | 110 | 123 | 31 | 22/20 | 6/5 |
| II | Standard | 320 | 76 | 45 | 14 | 6/6 | 2/2 |
| | Agro-Preculture | 400 | 104 | 61 | 26 | 39/37 | 10/9 |

| | | | | | | | |
|---|---|---|---|---|---|---|---|
| a: Number of shoots / number of explants having shoots | | | | | | | |

The experiment surprisingly demonstrates, that pre-cultivation for about 7 days significantly increases regeneration efficiency.

### REFERENCES

The references listed below and all references cited herein are incorporated herein by reference to the extent that they supplement, explain, provide a background for, or teach methodology, techniques, and/or compositions employed herein.
1. An et al. (1985) EMBO J 4:277-287
2. Atanassova et al. (1992) Plant J 2(3): 291-300
3. Ausubel FM et al. (1987) Current Protocols in Molecular Biology, Greene Publishing Assoc. and Wiley Interscience
4. Baker et al. (1987) EMBO J 6: 1547-1554
5. Bäumlein H et al. (1991a) Mol Gen Genet 225(3):459-467
6. Bäumlein H et al. (1991b) Mol Gen Genet 225:121-128
7. Becker et al. (1992) Plant Mol. Biol. 20: 49
8. Benfey et al.(1989) EMBO J 8:2195-2202
9. Bevan et al. (1984) Nucl Acid Res 12,8711-8720
10. Bruce et al. (1989) Proc Natl Acad Sci USA 86:9692-9696
11. Bustos MM et al. (1989) Plant Cell 1(9):839-53)
12. Chen and Winans (1991) J. Bacteriol. 173: 1139-1144
13. Christensen et al. (1989) Plant Mol. Biol. 12: 619-632
14. Christensen et al. (1992) Plant Mol Biol 18:675-689
15. Chui WL et al. (1996) Curr Biol 6:325-330;
16. Dale & Ow (1991) Proc Nat'l Acad Sci USA 88:10558-10562
17. de Block et al. (1987) EMBO J 6:2513-2518
18. De Block et al. (1989) Plant Physiol 91:694-701
19. Deblaere et al. (1985) Nucl Acids Res 13:4777-4788
20. Della-Cioppa et al. (1987) Bio/Technology 5:579-584
21. Dellaporta et al. (1988) In: Chromosome Structure and Function: Impact of New Concepts, 18th Stadler Genetics Symposium, 11:263-282
22. Dietert MF et al. (1982) Plant Science Letters 26:233-240
23. Dunwell JM (2000) J Exp Bot 51 Spec No:487-96
24. Eichholtz et al. (1987) Somatic Cell and Molecular Genetics 13, 67-76
25. EP-A1 0 120 516
26. EP-A1 0 333 033
27. EP-A1 0 335 528
28. EP-A1 0 388 186
29. EP-A1 0 807 836
30. EP-A1 270 615
31. Fedoroff NV & Smith DL (1993) Plant J 3:273- 289
32. Fire A. et al (1998) Nature 391:806-811
33. Fraley et al. (1983) Proc Natl Acad Sci USA 80:4803
34. Franck et al. (1980) Cell 21:285-294
35. Gallie et al. (1987) Nucl Acids Res 15:8693-8711
36. Gardner et al. (1986) Plant Mol Biol 6:221-228
37. Gatz et al. (1991) Mol Gen Genetics 227:229-237
38. Gatz et al. (1992) Plant J 2:397-404
39. Gatz et al. (1994) Mol Gen Genetics 243:32-38
40. Gatz et al. (1997) Annu Rev Plant Physiol Plant Mol Biol 48:89-108
41. Gelvin et al. (Eds) (1990) Plant Molecular Biology Manual; Kluwer Academic Publisher, Dordrecht, The Netherlands
42. GenBank Acc. No. X03677
43. GenBank Acc. No.: U38846, nucleotides 3862 to 5325 or else 5342
44. Gleave AP et al. (1999) Plant Mol Biol. 40(2):223-35
45. Glimelius K (1984) Physiol Plant 61:38-44
46. Gruber et al. (1993) ''Vectors for Plant Transformation," in METHODS IN PLANT MOLECULAR BIOLOGY AND BIOTECHNOLOGY; pp.89-119.
47. Guerrero et al. (1993) Mol Gen Genet 224:161-168
48. Hajdukiewicz et al. (1994) Plant Mol Biol 25:989-994
49. Hansen et al. (1994) Proc. Natl. Acad. Sci. USA 91:7603-7607
50. Haseloff et al.(1997) Proc Natl Acad Sci USA 94(6):2122-2127;
51. Hayford et al., Plant Physiol. 86:1216 (1988),
52. Hershey et al. (1991) Mol Gen Genetics 227:229-237
53. Hiei et al. (1994) Plant J 6: 271-282
54. Hille et al., Plant Mol. Biol. 7:171 (1986)
55. Hoekema (1985) In: The Binary Plant Vector System, Offsetdrukkerij Kanters B.V., Alblasserdam, Chapter V
56. Hoekema et al. (1983) Nature 303:179-181
57. Holsters et al. (1978) Mol Gen Genet 163:181-187
58. Holtorf S et al. (1995) Plant Mol Biol 29:637-649
59. Hood et al. (1986) J Bacteriol 168:1291-1301
60. Horsch RB et al. (1985) Science 225: 1229f
61. Ishida Y et al. (1996) Nature Biotech 745-750
62. Jarchow et al. (1991), Proc. Natl. Acad. Sci. USA 88:10426-10430
63. Jefferson (1987b) Plant Mol. Bio. Rep., 5:387-405
64. Jefferson et al. (1987a) EMBO J., 6:3901-3907
65. Jenes B et al.(1993) Techniques for Gene Transfer, in: Recombinant Plants, Vol. 1, Engineering and Utilization, edited by SD Kung and R Wu, Academic Press, pp. 128-143
66. Jones et al., Mol. Gen. Genet., 210:86 (1987),
67. Joseffson LG et al. (1987) J Biol Chem 262:12196-12201
68. Kado (1991) Crit Rev Plant Sci 10:1
69. Klapwijk et al. (1980) J. Bacteriol., 141,128-136
70. Koncz & Schell (1986) Mol Gen Genet 204:383-396
71. Koprek T et al. (1999) Plant J 19(6): 719-726
72. Last DI et al. (1991) Theor. Appl. Genet. 81, 581-588.
73. Lawson et al. (1994) Mol Gen Genet 245:608-615
74. Lazzeri A (1984) Annals of Botany 54:341-350
75. Leffel SM et al. (1997) Biotechniques. 23(5):912-8
76. Lepetit et al., Mol. Gen. Genet. 231: 276-285 (1992)
77. Li et al. (1992) Plant Mol Biol 20:1037-1048
78. Li LC (1982) Plant Cell Rep 1:209-211
79. Ludwig et al. (1990) Science 247:449
80. Lysnik et al. (1993) NAR 21:969-975
81. Maniatis T, Fritsch EF and Sambrook J (1989) Molecular Cloning: A Laboratory Manual, 2nd Ed., Cold Spring Harbor Laboratory, Cold Spring Harbor (NY)
82. Matzke MA et al. (2000) Plant Mol Biol 43:401-415
83. McElroy et al., Plant Cell 2:163171 (1990)
84. Mett et al. PNAS 90: 4567-4571 (1993)
85. Miki et al. (1993) "Procedures for Introducing Foreign DNA into Plants" in METHODS IN PLANT MOLECULAR BIOLOGY AND BIOTECHNOLOGY; pp.67-88
86. Millar et al. (1992) Plant Mol Biol Rep 10:324-414
87. Mol JN et al. (1990) FEBS Lett 268(2):427-430
88. Moloney et al., Plant Cell Reports 8: 238 (1989)
89. Mozo & Hooykaas, Plant Mol. Biol. 16 (1991), 917-918.
90. Murai et al., Science 23: 476-482 (1983)
91. Murashige T and Skoog F (1962) Physiol. Plant. 15, 472-497. (472/473)
92. Naested H (1999) Plant J 18:571-576
93. Nehra et al., Plant J., 5:285-297, 1994.
94. Odell et al. (1985) Nature 313:810-812
95. Odell et al. (1990) Mol Gen Genet 223:369-378
96. Olhoft PM et al. (2001) Plant Cell Rep 20: 706-711
97. Ow et al. (1986) Science 234:856-859;
98. Perera RJ et al. (1993) Plant Mol. Biol 23(4): 793-799;
99. Perl A et al. (1996) Nature Biotechnol 14: 624-628
100. Prasher et al. (1985) Biochem Biophys Res Commun 126(3):1259-1268)
101. Randez-Gil et al. (1995) Yeast 11:1233-1240
102. Reichel et al.(1996) Proc Natl Acad Sci USA 93(12):5888-5893;
103. Rouster J et al. (1998) Plant J 15:435-440
104. Sanger et al. (1977) Proc Natl Acad Sci USA 74:5463-5467
105. Sauer B (1998) Methods 14(4):381-92
106. Scheeren-Groot et al. (1994) J. Bacteriol 176: 6418-6426
107. Schena et al. (1991) Proc Nat'l Acad Sci USA 88:10421
108. Schenborn E, Groskreutz D. (1999) Mol Biotechnol 13(1):29-44
109. Schlaman HRM and Hooykaas PJJ (1997) Plant J 11:1377-1385
110. Sengupta-Gopalan et al., Proc. Nat'l Acad. Sci. USA 82: 3320-3324 (1985)
111. Shah et al. (1986) Science 233: 478
112. Sheehy et al. (1988) Proc Natl Acad Sci USA 85: 8805-8809;
113. Sheen et al.(1995) Plant J 8(5):777-784;
114. Shewmaker et al. (1985) Virology 140:281-288
115. Shirsat A et al. (1989) Mol Gen Genet 215(2):326-331
116. Silhavy TJ, Berman ML and Enquist LW (1984) Experiments with Gene Fusions, Cold Spring Harbor Laboratory, Cold Spring Harbor (NY)
117. Simpson et al. (1985) EMBO J 4:2723-2729
118. Stalberg K et al. (1996) Planta 199:515-519
119. Stougaard J; (1993) Plant J 3:755-761
120. Stringham GR (1979) Z Pflanzenphysiol 92:459-462
121. Svab et al., Plant Mol. Biol. 14:197 (1990<
122. The Maize Handbook, Chapter 116, Freeling and Walbot, Eds., Springer, New York (1994)
123. Tian et al. (1997) Plant Cell Rep 16:267-271;
124. Timko et al. (1985) Nature 318: 579-582
125. Twell et al. (1983) Sex. Plant Reprod. 6: 217-224
126. Twell et al. (1989b) Mol Gen Genet 217:240-245
127. US 4,801,340;
128. US 4,962,028
129. US 4,975,374)
130. US 4.940.838
131. US 5,225,341
132. US 5,352,605
133. US 5,504,200
134. US 5,565,350
135. US 5,608,152;
136. US 5,683,439
137. Van Laerebeke et al. (1974) Nature 252,169-170
138. Vanden Elzen et al. (1985) Plant Mol Biol. 5:299
139. Vasil et al.. (1992) Bio/Technology, 10:667-674
140. Velten J et al. (1984) EMBO J. 3(12): 2723-2730.
141. Vernade et al. (1988) J. Bacteriol. 170: 5822-5829
142. Wader et al., in TOMATO TECHNOLOGY 189-198 (Alan R. Liss, Inc. 1987)
143. Ward et al. (1993) Plant Mol Biol 22:361-366
144. Waterhouse PM et al. (1998) Proc Natl Acad Sci USA 95:13959-64
145. Watson et al. (1975) J. Bacteriol 123, 255-264
146. Watson et al. (1985) EMBO J 4(2):277- 284
147. Weeks et al. (1993) Plant Physiol 102:1077-1084
148. WO 00/15815
149. WO 02/00900
150. WO 84/02913
151. WO 91/13980
152. WO 91/13991
153. WO 93/01294
154. WO 93/21334
155. WO 95/15389
156. WO 95/19443
157. WO 95/23230
158. WO 98/45456
159. WO 98/45461
160. WO 99/16890
161. WO 00/44895;
162. WO 00/49035;
163. WO 00/63364
164. WO 00/68374;
165. WO 97/41228;
166. WO 99/32619
167. WO 99/53050;
168. Zupan et al. (2000) Plant J 23(1):11-28

## Claims

1. A method for producing a transgenic Brassica plant comprising the steps of:
(a) isolating cotyledonary petioles from germinated seeds of a Brassica plant,
(b) treating said petioles with an Agrobacterium solution, wherein said Agrobacterium comprises a T-DNA comprising a selectable marker gene and - optionally - an additional plant expression cassette for an agronomically valuable trait,
(c) transferring said treated petioles on a co-cultivation medium comprising at least one plant growth regulator solidified with an agar, said agar **characterized by** an electroendosmosis value (Mr) of below 0.5, a content of calcium ions of less then 1000 ppm, and a content of magnesium ions of less then 500 ppm, and culturing said petioles on said co-cultivation medium for about 1 to about 5 days,
(d) transferring said co-cultivated petioles on a regeneration and selection medium comprising at least one plant growth regulator, at least one antibiotic suitable to inhibit Agrobacterium growth, and at least one compound which in combination with the selectable marker gene of (a) allows for identification and/or selection of plant cells, organs or plants comprising said selectable marker gene, said medium solidified with an agar, said agar **characterized by** an electroendosmosis value (Mr) of below 0.5, a content of calcium ions of less then 1000 ppm, and a content of magnesium ions of less then 500 ppm,
(e) cultivating said petioles on said co-cultivation and regeneration medium until shoots are induced and developed from said petioles and isolating said shoots from said petioles,
(f) transferring said isolated shoots on a shoot elongation and regeneration medium comprising at least one plant growth regulator and a compound which in combination with the selectable marker gene of (a) allows for identification and/or selection of plant cells, organs or plants comprising said selectable marker gene, said medium solidified with an agar, said agar **characterized by** an electroendosmosis value (Mr) of below 0.5, a content of calcium ions of less then 1000 ppm, and a content of magnesium ions of less then 500 ppm, wherein the agar content in said medium is between about 0.3% and about 0.6% (w/w) agar, and wherein said medium comprises between about 1% and 2.5% (w/w) sucrose,
(g) cultivating said isolated shoots on said shoot elongation and regeneration medium until said shoots have formed roots, and further regenerating the so derived plantlets into mature plants, which comprise inserted into their genome a T-DNA comprising a selectable marker gene and - optionally - an additional plant expression cassette for an agronomically valuable trait.

2. The method of claim 1, wherein the Agrobacterium strain is not a supervirulent strain.

3. The method of claim 1 or 2, wherein the Agrobacterium strain is a nopaline-type strain.

4. The method of any of Claim 1 to 3, wherein the petioles are isolated from seedlings germinated for not longer then 5 days.

5. The method of any of Claim 1 to 4, wherein the media of at least one of step (c), (d), and/or (e) comprises between about 1 mg/l and about 10 mg/l 6-benzylaminopurine (BAP).

6. The method of any of Claim 1 to 5, wherein the media of at least one of step (c), (d), and/or (e) comprises between about 0.1 mg/l and about 2 mg/l Gibberellic acid (GA3).

7. The method of any of Claim 1 to 6, wherein the media of at least one of step (f) and/or (g) comprises between about 0.001 mg/l and about 0.1 mg/l 6-benzylaminopurine (BAP) and between about 0.01 mg/l and about 1 mg/l indole butyric acid (IBA).
